# EUROPÄISCHE PATENTANMELDUNG

(11) **EP 4 595 913 A1**
(43) Veröffentlichungstag der Anmeldung: **06.08.2025**
(21) Anmeldenummer: 25150472.6
(22) Anmeldetag: 07.01.2025
(51) Int. Cl.: A61B 18/14, A61B 18/00

(54) **ELEKTROCHIRURGISCHES HANDINSTRUMENT UND ELEKTRODE FÜR EIN ELEKTROCHIRURGISCHES HANDINSTRUMENT**

(30) Priorität: 05.02.2024 DE 102024103121
(71) Anmelder: Olympus Winter & Ibe GmbH, 22045 Hamburg (DE)
(72) Erfinder: KNOPF, Christoph, 23617 Stockelsdorf (DE); Brockmann, Christian, 21279 Hollenstedt (DE); Lavicka, Jiri, 75002 Prerov (CZ)
(74) Vertreter: Hoener, Matthias

(57) **Zusammenfassung**

Die Erfindung schafft eine Elektrode sowie ein elektrochirurgisches Handinstrument, das gleichermaßen eine hohe chemische Resistenz und eine hohe Temperaturfestigkeit aufweist. Das wird dadurch erreicht, dass eine Elektrode (10) für das elektrochirurgische Handinstrument im Wesentlichen aus einem elektrisch leitenden Draht besteht, wobei dieser Draht mit seinen beiden Enden (25, 26) mit einem Elektrodenträger (14) des Handinstrumentes gekoppelt ist. Dieser Draht setzt sich aus mindestens zwei verschiedenartigen Materialien zusammen. Dabei weist eines dieser Materialien M₁ eine Schmelztemperatur T₁ auf und das zweite Material M₂ eine Schmelztemperatur T₂. Die Schmelztemperatur T₂ des Materials M₂ ist größer als die Schmelztemperatur T₁ des Materials M₁.

## Beschreibung

Die Erfindung betrifft eine Elektrode für ein elektrochirurgisches Handinstrument gemäß dem Oberbegriff des Anspruchs 1 sowie ein elektrochirurgisches Handinstrument gemäß Anspruch 15.

Elektrochirurgische Handgeräte, insbesondere Resektoskope, der gattungsgemäßen Art werden vor allem in der Urologie bei elektrochirurgischen Arbeiten verwendet. Dabei kommen diese Geräte üblicherweise zur Resektion und zur Evaporation von Gewebe, zum Beispiel von Gewebe im unteren Harntrakt, zum Einsatz. Dazu kann das Handgerät, insbesondere das Resektoskop, einen längs verschiebbaren Elektrodenträger aufweisen, der nach der Einführung des Geräts in den zu behandelnden Körper mit einem distalen Arbeitsende aus einem distalen Ende des Instrumentenschafts des Handgeräts vorgeschoben werden kann. An dem Elektrodenträger ist an einem distalen Ende eine elektrochirurgische Elektrode angeordnet. Diese Elektrode kann beispielsweise die Form einer Schlinge aufweisen und wird zur Manipulation des Gewebes je nach Bauart des Instrumentes durch das Gewebe gezogen oder gedrückt.

Für die oben genannte Anwendung wird die Elektrode mit einem hochfrequenten elektrischen Strom beaufschlagt. Bekannte Elektroden für diese Hochfrequenzchirurgie werden aus einem homogenen Metall gefertigt. Dabei wird zwischen zwei Gruppen unterschieden, nämlich zwischen Elektroden aus Metallen mit einem hohen Schmelzpunkt, wie beispielsweise Wolfram, und Elektroden aus Edelmetallen, wie beispielsweise Platin oder Platinlegierungen. Der Vorteil der Verwendung von Elektroden aus Metallen mit einer hohen Schmelztemperatur besteht darin, dass sie auch bei hohen Temperaturen zuverlässig funktionieren und nicht brechen bzw. ihre Form verändern. Allerdings sind insbesondere Elektroden aus Wolfram weniger zuverlässig gegenüber chemischen Beanspruchungen, wodurch die Lebensdauer der Elektroden reduziert wird. Insbesondere bei der Verwendung eines Plasmas erweist sich dies als nachteilig.

Edelmetalle weisen hingegen gerade diese chemische Resistenz auf, besitzen hingegen eine geringere Schmelztemperatur als beispielsweise Wolfram, was sich insbesondere in Situationen, in denen eine höhere Stromdichte erreicht wird, als nachteilig erweisen kann. So kann es beispielsweise vorkommen, dass während der Behandlung eines Patienten, die mit einer hochfrequenten Spannung beaufschlagte Elektrode in die Nähe eines in dem Körper des Patienten vorhandenen elektrisch leitenden Implantats kommt. Bei einer eventuellen Funkenentladung entstehen hohe elektrische Ströme, die dazu führen können, dass die Elektrode aus Platin schmilzt bzw. sich derart in ihrer Form verändert, dass sie ausgetauscht werden muss. Demnach existieren zurzeit zwei gängige Varianten für Elektroden, die beide ihre Vor- und Nachteile aufweisen, weswegen es keine ideale Elektrode gibt, welche die genannten Nachteile nicht aufweist.

Der Erfindung liegt die Aufgabe zugrunde, eine Elektrode sowie ein elektrochirurgisches Handinstrument zu schaffen, das gleichermaßen eine hohe chemische Resistenz und eine hohe Temperaturfestigkeit aufweist.

Eine Lösung dieser Aufgabe wird durch die Merkmale des Anspruchs 1 beschrieben. Demnach ist es vorgesehen, dass eine Elektrode für ein elektrochirurgisches Handinstrument, bei dem es sich beispielsweise um ein Resektoskop handeln kann, im Wesentlichen aus einem elektrisch leitenden Draht besteht, wobei dieser Draht mit seinen beiden Enden mit einem Elektrodenträger des Handinstrumentes gekoppelt ist. Dieser Draht setzt sich aus mindestens zwei verschiedenartigen Materialien zusammen. Dabei weist eines dieser Materialien M₁ eine Schmelztemperatur T₁ auf und das zweite Material M₂ eine Schmelztemperatur T₂. Die Schmelztemperatur T₂ des Materials M₂ ist größer als die Schmelztemperatur T₁ des Materials M₁. Durch diese unterschiedlichen Eigenschaften bzw. Schmelztemperaturen der mindestens zwei Materialien lassen sich die oben genannten Probleme beheben. Es ist mit dieser Konfiguration möglich, eine Elektrode zu schaffen, die sowohl chemisch stabil ist und gleichzeitig eine hohe Stromfestigkeit aufweist, d. h. auch bei hohen Stromdichten mechanisch stabil bleibt.

Ein weiteres vorteilhaftes Ausführungsbeispiel der Erfindung sieht es vor, dass das Material M₁ eine Plasmabeständigkeit bzw. ein Standardpotential P₁ und das Material M₂ eine Plasmabeständigkeit bzw. ein Standardpotential P₂ aufweisen. Dabei ist es erfindungsgemäß vorgesehen, dass P₁ größer ist als P₂. Die Plasmabeständigkeit bzw. das Standardpotential der Materialien verhält sich somit gerade entgegengesetzt zueinander, wie die Schmelztemperatur der beiden Materialien. Diese Wahl der Materialien stellt eine weitere Möglichkeit dar, eine Elektrode zu schaffen, die chemisch stabil ist und gleichzeitig eine hohe Stromfestigkeit aufweist.

Ein bevorzugtes Ausführungsbeispiel der Erfindung kann es vorsehen, dass das Material M₂ einen Kern des Drahtes bildet und das Material M₁ eine den Kern umhüllende äußere Schicht bildet oder dass das Material M₁ einen Kern des Drahtes bildet und das Material M₂ eine den Kern umhüllende äußere Schicht bildet. Durch diese Anordnung der beiden Materialien lässt sich zum einen erreichen, dass die Elektrode nach außen hin chemisch stabil ist und zum anderen gleichzeitig auch bei hohen Temperaturen ihre Form nicht verändert. Je nach Anwendung bzw. Anforderung können so verschiedene Ausführungsformen der Elektrode verwendet werden.

Ein weiteres denkbares Ausführungsbeispiel der Erfindung kann es vorsehen, dass sich der Draht schalenartig aus mehreren Schichten der Materialien M₁ und M₂ zusammensetzt, wobei sich die Materialien M₁ und M₂ gegenseitig umschließen und der Kern des Drahtes aus dem Material M₁ oder M₂ gebildet ist. Durch diesen zwiebelartigen Aufbau der Elektrode lässt sich gleichsam eine hohe chemische Stabilität und gleichzeitig eine hohe Temperaturfestigkeit der Elektrode erreichen.

Es kann darüber hinaus erfindungsgemäß vorgesehen sein, dass sich die Kern-Schicht-Struktur des Drahtes aus den mindestens zwei Materialien M₁ und M₂ über die gesamte Länge des Drahtes erstreckt. Diese Form der Elektrode gestaltet sich insbesondere für die Herstellung des Drahtes aus zwei Komponenten als vorteilhaft, da dieser kostengünstig in größeren Mengen herstellbar ist. So ist es beispielsweise denkbar, dass das eine Material von dem anderen von einem Rohr umschlossen wird oder mit dem entsprechenden Material bedampft wird. Auch andere Möglichkeiten der Herstellung kommen in Betracht.

Ein vorteilhaftes Ausführungsbeispiel der Erfindung kann es vorsehen, dass sich der Draht aus mindestens zwei Stücken zusammensetzt, nämlich einem ersten Endstück E₁ und mindestens einem Mittelstück MST, wobei das Mittelstück MST an dem Endstück E₁ angeordnet ist und das Endstück E₁ mit dem Elektrodenträger des Handinstrumentes koppelbar ist. Durch diese Bauart der Elektrode in verschiedene Abschnitte bzw. Teile lässt sich ein besonders vorteilhaftes Design erreichen, bei dem eine Elektrode geschaffen wird, die sowohl chemisch stabil, hitzeresistent und günstig in der Herstellung ist.

Ein besonders vorteilhaftes Ausführungsbeispiel der Erfindung kann es vorsehen, dass sich der Draht aus mindestens drei Stücken zusammensetzt, nämlich einem ersten Endstück E₁ und einem zweiten Endstück E₂ und mindestens einem Mittelstück MST, wobei das Mittelstück MST zwischen den beiden Endstücken E₁ und E₂ angeordnet ist und die Endstücke E₁ und E₂ mit dem Elektrodenträger des Handinstrumentes koppelbar sind. Durch diese Aufteilung der Elektrode in verschiedene Abschnitte bzw. Teile lässt sich ein besonders vorteilhaftes Design erreichen, bei dem eine Elektrode geschaffen wird, die sowohl chemisch stabil, hitzeresistent und günstig in der Herstellung ist.

Insbesondere ist es vorgesehen, dass das Mittelstück MST des Drahtes einen Kern aus dem Material M₂ und eine umhüllende äußere Schicht aus dem Material M₁ aufweist oder dass das Material M₁ den Kern des Mittelstücks MST bildet und das Material M₂ die umhüllende äußere Schicht. Durch die Beschränkung der Materialien M₁ und M₂ sowie insbesondere die Beschränkung dieser speziellen Kern-Schicht-Struktur auf das Mittelstück MST lässt sich ein erhebliches Maß an Material und somit Kosten einsparen. Die Verwendung der Materialien beschränkt sich durch dieses Design auf den Bereich, der tatsächlich mit dem Körpergewebe in Kontakt kommt. Die Endstücke E₁ und E₂, die nicht mit dem Gewebe bzw. dem Plasma in Kontakt kommen bzw. nicht dazu dienen, das Gewebe gezielt zu manipulieren, können aus einem anderen, günstigeren und besser handhabbaren Material gefertigt werden.

Bevorzugt ist es denkbar, dass sich das Mittelstück MST des Drahtes schalenartig aus mehreren Schichten der Materialien M₁ und M₂ zusammensetzt, wobei sich die Materialien M₁ und M₂ gegenseitig umschließen und der Kern des Drahtes aus dem Material M₁ oder M₂ gebildet ist. Durch dieses spezielle Ausführungsbeispiel lässt sich eine Elektrode schaffen, die sowohl günstig in der Herstellung, chemisch stabil sowie hitzeresistent ist.

Das Endstück E₁ ist bzw. die Endstücke E₁ und E₂ sind aus einem elektrisch leitfähigen Material M₃ gefertigt und mit ihren Enden E₁ und E₂ mit dem Mittelstück MST elektrisch leitend oder isolierend sowie mechanisch verbunden. Eine Möglichkeit der Verbindung der Endstücke E₁ und/oder E₂ mit dem Mittelstück MST besteht im Verkleben, Verschweißen, Verklemmen oder einer Vercrimpung mittels einer Hülse. Diese Hülse kann auch mit den beiden Endstücken E₁ und/oder E₂ verschweißt werden, wobei sich im Inneren der Hülse der Kern gemäß den vorherigen Ausführungsbeispielen befindet. Durch die hier genannten Möglichkeiten der Verbindung wird eine Elektrode geschaffen, die sich aus mehreren Teilen zusammensetzen lässt, aber gleichsam die notwendige chemische sowie mechanische Festigkeit aufweist. Bei diesem Ausführungsbeispiel der Elektrode, bestehend aus zwei oder drei getrennten Teilen, ist es denkbar, dass das Mittelstück MST aus den Materialien M₁ oder M₂ und die Hülse komplementär dazu aus den Materialien M₂ oder M₁ ausgebildet ist. Außerdem ist es für das Ausführungsbeispiel der Elektrode, bei dem eines der Endstücke elektrisch isolierend mit dem Mittelstück verbunden ist, denkbar, dass dieses Endstück aus einem elektrisch isolierenden Material hergestellt ist.

Der hier beschriebene Draht kann einen Durchmesser von 0,2 mm bis 1 mm, vorzugsweise von 0,28 mm, aufweisen. Gleichermaßen ist es denkbar, dass der Draht andersartig dimensioniert ist. Darüber hinaus kann es vorgesehen sein, dass die den Kern umhüllende äußere Schicht oder die Hülse eine Schichtdicke von 3 µm bis 0,2 mm, insbesondere 0,01 mm bis 0,1 mm, vorzugsweise 0,05 mm, aufweist.

Als besonders vorteilhaft hat sich erwiesen, dass es sich bei dem Material M₁ um Platin, oder um eine Platinlegierung, wie beispielsweise Platin-Iridium oder Platin-Wolfram, handelt. Bei dem Material M₂ kann es sich um Wolfram, Tantal oder Molybdän handeln. Schließlich ist es denkbar, dass es sich bei dem Material M₃ um Stahl bzw. Edelstahl, Kupfer oder eine Kupferlegierung oder einen elektrischen Isolator handelt. Dabei sei ausdrücklich darauf hingewiesen, dass diese Aufzählung nicht abschließend ist, sondern es vielmehr vorgesehen ist, dass auch andere Materialien mit vergleichbaren Eigenschaften verwendet werden.

Ein elektrochirurgisches Handinstrument zur Lösung der genannten Aufgabe weist die Merkmale des Anspruchs 15 auf. Demnach ist es vorgesehen, dass dieses elektrochirurgische Handinstrument, bei dem es sich beispielsweise um ein Resektoskop handeln kann, eine Elektrode nach mindestens einem der Ansprüche 1 bis 14 aufweist.

Ein bevorzugtes Ausführungsbeispiel der Erfindung wird nachfolgend anhand der Zeichnung näher beschrieben. In dieser zeigen:
- Fig. 1: eine schematische Darstellung eines chirurgischen Handgeräts, insbesondere eines Resektoskops,
- Fig. 2: eine Seitenansicht einer Elektrode,
- Fig. 3: eine Frontdarstellung der Elektrode gemäß Fig. 2,
- Fig. 4: ein Schnitt durch ein erstes Ausführungsbeispiel der Elektrode,
- Fig. 5: ein Schnitt durch ein weiteres Ausführungsbeispiel der Elektrode,
- Fig. 6: ein Schnitt durch ein weiteres Ausführungsbeispiel der Elektrode,
- Fig. 7: eine Seitenansicht eines weiteren Ausführungsbeispiels der Elektrode, und
- Fig. 8: eine Darstellung eines weiteren Ausführungsbeispiels der Elektrode.

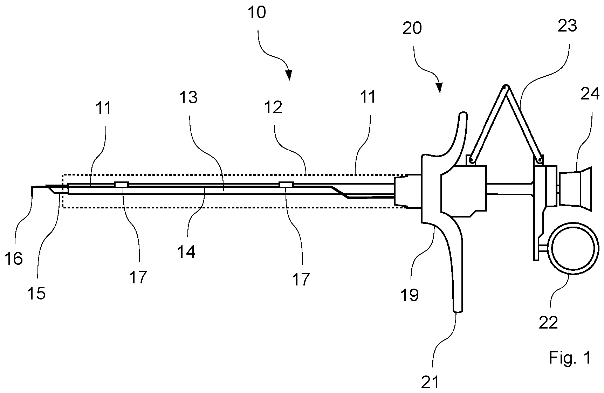

Fig. 1 zeigt eine schematische, seitliche Schnittdarstellung eines Resektoskopes 10. Das Resektoskop 10 weist einen Resektoskopschaft 11 auf, der einen dargestellten äußeren Schaft 12 bzw. ein Hüllrohr umfasst. Innerhalb des äußeren Schaftes 12 verläuft ein rohrartiger innerer Schaft 13. Innerhalb des inneren Schaftes 13 sind ein Elektrodenträger 14 sowie eine angedeutete Optik 15 dargestellt. Darüber hinaus können weitere hier nicht dargestellte Elemente in dem Resektoskop 10 angeordnet sein, wie beispielsweise ein separates Spülrohr und dergleichen.

Der Elektrodenträger 14 weist an einem distalen Ende ein elektrochirurgisches Werkzeug bzw. eine Elektrode 16 auf. Die hier dargestellte Elektrode 16 ist als Schlinge ausgebildet.

Der Elektrodenträger 14 kann durch Betätigung eines Handgriffs 19 zwangsgeführt axial in distale und proximale Richtung bewegt werden. Dabei kann er über das distale Ende des inneren Schaftes 13 und des äußeren Schaftes 12 hinausgeschoben werden. So wird es dem Operateur ermöglicht auch weiter von der Resektoskopspitze entferntes Gewebe zu manipulieren. Zu diesem Zweck können ferner der innere Schaft 13 und/oder der Elektrodenträger 14 um ihre Längsachse drehbar gelagert sein. Für die Manipulation des Gewebes wird die Elektrode 16 mit einem hochfrequenten elektrischen Strom beaufschlagt.

Das in der Fig. 1 dargestellte Resektoskop 10 weist einen passiven Transporteur auf, bei dem ein Schlitten 20 durch Relativbewegung der proximal an dem Resektoskopschaft 11 angeordneten Griffteilen 21 und 22 gegen eine von einer Federbrücke 23 aufgebrachte Federkraft in distale Richtung gegen das distale, erste Griffteil 21 verschoben wird. Bei der Verschiebung des Schlittens 20 in distale Richtung gegen das Griffteil 21 wird der Elektrodenträger 14 in nicht dargestellter Weise in distale Richtung verschoben. Bei einer Entlastung der Handgriffteile 21, 22 zwingt die von der Federbrücke 23 erzeugte Federkraft den Schlitten 20 zurück in seine Ausgangsstellung, wobei der Elektrodenträger 14 in proximale Richtung gezogen wird. Bei der Rückverschiebung des Schlittens 20 kann ohne Handkraft des Operateurs, also passiv, ein elektrochirurgischer Eingriff mit der Elektrode 16 vorgenommen werden.

Für die gezielte Behandlung mittels der Elektrode 16 ist die Optik 15 derart positioniert, dass der Operateur einen optimalen Blick auf den Bereich der Operation erhält. Dazu weist das Resektoskop 10 an einem proximalen Ende ein Okular 24 auf, welches mit der Optik 15 verbunden ist. Alternativ ist es auch denkbar, dass anstelle des Okulars 24 eine Kamera an dem Resektoskop 10 angeordnet ist.

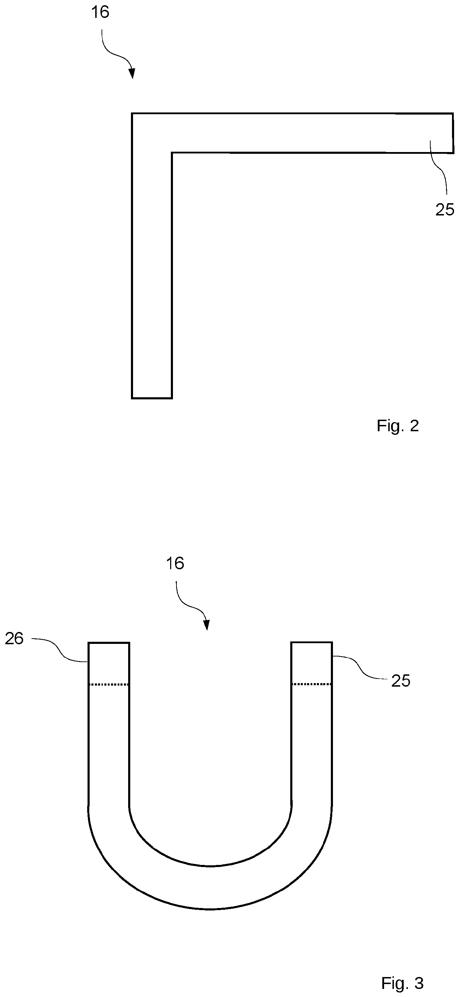

Die hier beschriebene Elektrode 16 weist die Form einer Schlinge auf. Eine derartige Schlingenstruktur ist stark schematisiert in den Fig. 2 und 3 dargestellt. Dabei ist ein Draht derart zu einer U-förmigen Schlinge geformt, dass er mit den beiden Enden 25 und 26 mit dem Elektrodenträger 14 des Resektoskopes 10 elektrisch und gegebenenfalls auch mechanisch koppelbar ist. Die Kopplung mit dem Elektrodenträger 14 erfolgt zumeist durch eine Steckverbindung. Es sei allerdings ausdrücklich darauf hingewiesen, dass auch andere Elektrodenformen, wie bei beispielsweise Knopfelektroden oder Nadelelektroden, denkbar sind.

Damit die Elektrode 16 auch bei großer Hitze, d. h. bei großer Stromdichte, die bei der Beaufschlagung der Elektrode 16 mit einer hochfrequenten Wechselspannung auftreten kann, ihre Form beibehält bzw. nicht beschädigt wird, ist es üblich, den Draht bzw. die Elektrode 16 aus Wolfram herzustellen. Wolfram zeichnet sich bekanntermaßen durch seine extrem hohe Schmelztemperatur aus. Gleichermaßen ist es erforderlich, dass die Elektrode 16 gegenüber chemischen Reaktionen, die insbesondere bei der Plasma-Anwendung auftreten können, formstabil bleibt, um so möglichst über einen langen Zeitraum ohne Einschränkung der Leistung verwendet werden zu können. Durch die Verwendung von Platin oder einer Platinlegierung als Material für den Draht kann dies erreicht werden. Allerdings ist Platin zum einen nicht besonders hitzeresistent und zum anderen sehr teuer.

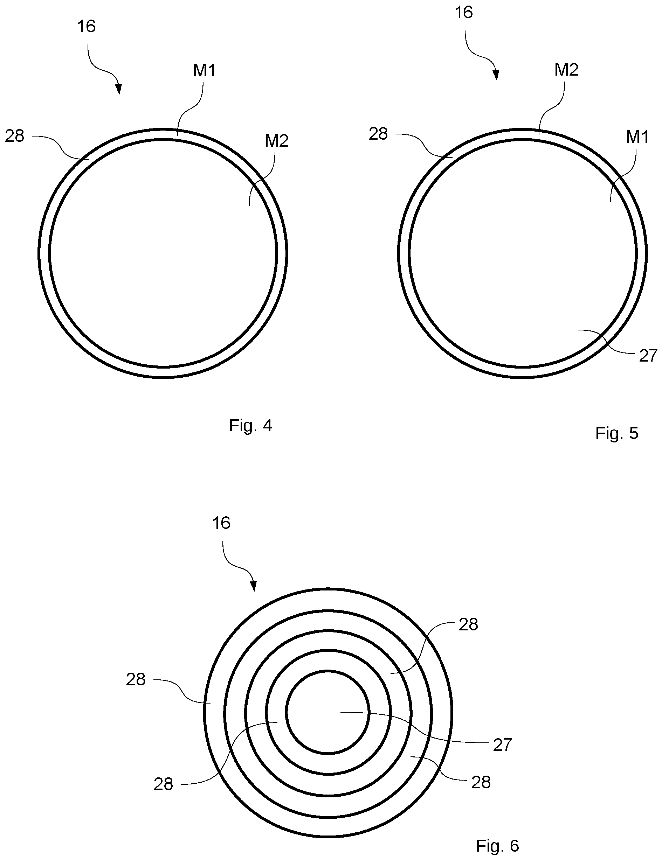

Die hier beschriebene Elektrode 16 besteht mindestens aus zwei verschiedenen Materialien. Die Fig. 4 zeigt schematisiert einen Querschnitt eines möglichen Ausführungsbeispiels der Elektrode 16. Demnach ist es vorgesehen, dass ein Kern 27 der Elektrode 16 durch ein Material M₂ und eine den Kern 27 umhüllende äußere Schicht 28 durch ein Material M₁ gebildet wird. Dabei weist das Material M₂ die Schmelztemperatur T₂ und das Material M₁ die Schmelztemperatur T₁ auf. Ein besonders bevorzugtes Ausführungsbeispiel sieht es vor, dass die Temperatur T₂ größer ist als die Temperatur T₁. Bei dem Material M₂ könnte es sich demnach beispielsweise um Wolfram, Tantal oder Molybdän handeln, die eine hohe Schmelztemperatur aufweisen. Im Gegensatz dazu könnte es sich bei dem Material M₁ um Metalle mit einer geringeren Schmelztemperatur, wie beispielsweise Platin, einer Platinlegierung, wie beispielsweise Platin-Iridium oder Platin-Wolfram, handeln. Durch diese Konfiguration hätte die Elektrode 16 eine hohe chemische Resistenz gegenüber äußeren Einflüssen und wäre gleichzeitig hitzebeständig gegenüber hohen Stromdichten. Alternativ ist es für entsprechende Anwendungen auch denkbar, dass die Anordnung der genannten Materialien M₂ und M₁ genau andersrum sind. Dies ist beispielhaft durch das Ausführungsbeispiel der Fig. 5 dargestellt. Dort wird die äußere Schicht 28 durch das Material M₂ und der Kern 27 durch das Material M₁ gebildet.

Ein weiteres mögliches Ausführungsbeispiel der Erfindung wird durch die Fig. 6 dargestellt. Hier umhüllen sich zwiebelschalenartig ausgehend von einem Kern 27 mehrere aufeinanderfolgende Schichten 28. Je nach Anwendung bzw. Anforderung können hier die Materialien M₁ und M₂ nahezu beliebig angeordnet werden. Nur der Vollständigkeit halber sei erwähnt, dass es auch denkbar ist, dass mehr als zwei verschiedene Materialien für diese Kern-Schicht-Struktur verwendet werden können.

Bei den vorgenannten Ausführungsbeispielen erstreckt sich die Kern-Schicht-Struktur über die gesamte Länge des Drahtes, d. h. von dem Ende 25 bis zu dem Ende 26. In der Regel ist dieser Draht 20 mm bis 80 mm lang. Der Durchmesser des Drahtes kann 0,2 mm bis 1 mm, vorzugsweise 0,28 mm, betragen, während die umhüllende äußere Schicht 28 eine Schichtdicke von 3 µm bis 0,2 mm, insbesondere 0,01 mm bis 0,1 mm, vorzugsweise 0,05 mm, aufweisen kann.

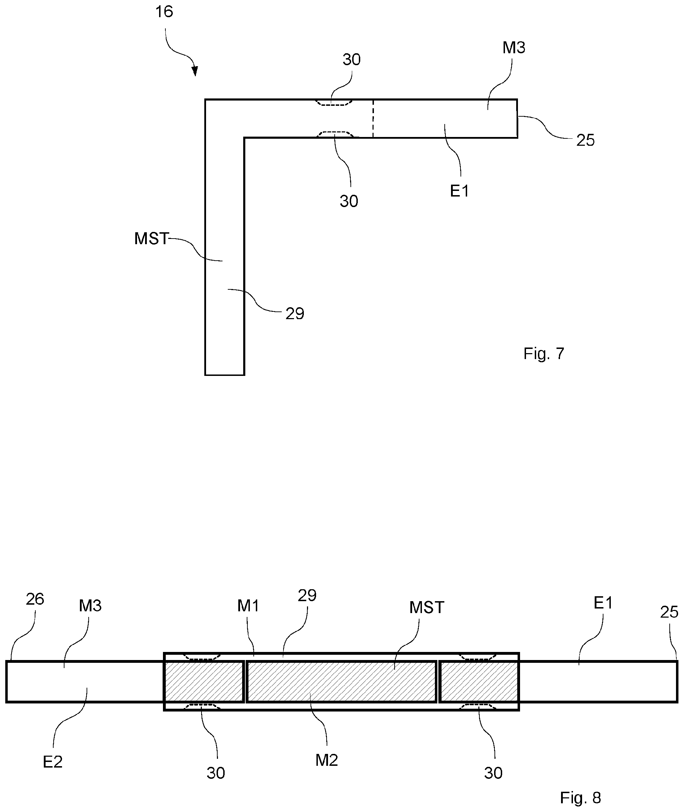

Ein weiteres Ausführungsbeispiel der Elektrode 16 ist in den Fig. 7 und 8 dargestellt. Die dort gezeigt Elektrode setzt sich aus drei Abschnitten bzw. Stücken zusammen, nämlich einen ersten Endstück E₁, einem zweiten Endstück E₂ sowie einem Mittelstück MST. Das Mittelstück MST ist zwischen den beiden Endstücken E₁ und E₂ angeordnet. Die beiden Endstücke E₁ und E₂ weisen die beiden Enden 25 und 26 auf, mit denen sie auf die oben beschriebene Art und Weise an den Elektrodenträger 14 koppelbar sind.

Ein Ausführungsbeispiel kann es vorsehen, dass das Mittelstück MST der Elektrode 16 die gleiche Kern-Schicht-Struktur aufweist, wie zuvor für die anderen Ausführungsbeispiele beschrieben. Ein weiteres Ausführungsbeispiel sieht es vor, dass das Mittelstück MST aus dem Material M₁ oder M₂ gebildet ist. Die Endstücke E₁ und E₂ wiederum sind aus Stahl oder Edelstahl gefertigt, sodass sich zwischen den beiden Endstücken E₁ und E₂ ein elektrischer Leiter aus Wolfram, Tantal oder Molybdän oder aus Platin, einer Platinlegierung, wie beispielsweise Platin-Iridium oder Platin-Wolfram, befindet. Zur Fixierung des Mittelstücks MST zwischen den beiden Endstücken E₁ und E₂ ist es denkbar, dass die einzelnen Stücke miteinander verklebt, verschweißt, vercrimpt oder verklemmt sind. Bei dem in der Fig. 8 dargestellten Ausführungsbeispiel ist um das Mittelstück MST und die beiden Endstücke E₁ und E₂ wenigstens abschnittsweise eine Hülse 29 angeordnet. Wenn das Mittelstück MST aus dem Material M₁ gebildet ist, besteht die Hülse 29 aus dem Material M₂. Wird die Hülse 29 hingegen aus dem Material M₁ gebildet, besteht das Mittelstück MST aus dem Material M₂. Zum Befestigen der Hülse 29 an den Endstücken E₁ und E₂ ist es denkbar, dass die Hülse 29 mit den Endstücken E₁ und E₂ verschweißt oder vercrimpt wird. Dazu werden die Endstücke der Hülse 29 mit Einkerbungen 30 versehen, um die genannten Bestandteile der Elektrode 16 mechanisch stabil sowie elektrisch leitend miteinander zu verbinden. Es ist auch denkbar, dass die Teile miteinander verschweißt und zusätzlich vercrimpt werden, um auch gegenüber stärkeren mechanischen Belastungen die ausreichende Stabilität aufzuweisen. Es sei ausdrücklich darauf hingewiesen, dass die Dimensionierung und die Anordnung, insbesondere der Hülse 29, von der hier nur beispielhaft dargestellten abweichen kann. Ansonsten entsprechen die Dimensionen der Elektrode 16 den oben genannten Werten.

Das zuletzt genannte Ausführungsbeispiel vereint die Vorteile einer kostengünstigen Herstellung, einer extrem hohen Hitzebeständigkeit sowie einer chemischen Resistenz gegenüber den äußeren Einflüssen, die während der Behandlung auf die Elektrode 16 einwirken.

**Bezugszeichenliste:**

| | | | |
|---|---|---|---|
| 10 | Resektoskop | M₁ | Material |
| 11 | Resektoskopschaft | M₂ | Material |
| 12 | äußerer Schaft | E₁ | Endstück |
| 13 | innerer Schaft | E₂ | Endstück |
| 14 | Elektrodenträger | MST | Mittelstück |
| 15 | Optik | | |
| 16 | Elektrode | | |
| 17 | Führungselement | | |
| 18 | Längsachse | | |
| 19 | Handgriff | | |
| 20 | Schlitten | | |
| 21 | Griffteil | | |
| 22 | Griffteil | | |
| 23 | Federbrücke | | |
| 24 | Okular | | |
| 25 | Ende | | |
| 26 | Ende | | |
| 27 | Kern | | |
| 28 | Schicht | | |
| 29 | Hülse | | |
| 30 | Einkerbung | | |

## Patentansprüche

1. Elektrode (16) für ein elektrochirurgisches Handinstrument, insbesondere für ein Resektoskop (10), bestehend aus einem elektrisch leitenden Draht, wobei die beiden Enden (25, 26) des Drahtes mit einem Elektrodenträger (14) des Handinstruments koppelbar sind, **dadurch gekennzeichnet, dass** sich der elektrisch leitende Draht aus mindestens zwei verschiedenartigen Materialien zusammensetzt, nämlich mindestens einem Material M₁ mit einer Schmelztemperatur T₁ und mindestens einem Material M₂ mit einer Schmelztemperatur T₂, wobei T₂ größer ist als T₁.

2. Elektrode (16) für ein elektrochirurgisches Handinstrument nach Anspruch 1, **dadurch gekennzeichnet, dass** das Material M₁ eine Plasmabeständigkeit bzw. ein Standardpotential P₁ und das Material M₂ eine Plasmabeständigkeit bzw. ein Standardpotential P₂ aufweisen, wobei P₁ größer ist als P₂.

3. Elektrode (16) für ein elektrochirurgisches Handinstrument nach Anspruch 1 oder 2, **dadurch gekennzeichnet, dass** das Material M₂ einen Kern (27) des Drahtes bildet und das Material M₁ eine den Kern (27) umhüllende äußere Schicht (28) bildet oder dass das Material M₁ einen Kern (27) des Drahtes bildet und das Material M₂ eine den Kern (27) umhüllende äußere Schicht (28) bildet.

4. Elektrode (16) für ein elektrochirurgisches Handinstrument nach einem der vorherigen Ansprüche, **dadurch gekennzeichnet, dass** sich der Draht schalenartig aus mehreren Schichten (28) der Materialien M₁ und M₂ zusammensetzt, wobei sich die Materialien M₁ und M₂ gegenseitig umschließen und der Kern (27) des Drahtes aus dem Material M₁ oder M₂ gebildet ist.

5. Elektrode (16) für ein elektrochirurgisches Handinstrument nach einem der vorherigen Ansprüche, **dadurch gekennzeichnet, dass** sich die Kern-Schicht-Struktur des Drahtes bestehend aus den mindestens zwei Materialien M₁ und M₂ über die gesamte Länge des Drahtes erstreckt.

6. Elektrode (16) für ein elektrochirurgisches Handinstrument nach einem der vorherigen Ansprüche, **dadurch gekennzeichnet, dass** sich der Draht aus mindestens zwei Stücken zusammensetzt, nämlich einem ersten Endstück E₁ und mindestens einem Mittelstück MST, wobei das Mittelstück MST an dem Endstück E₁ angeordnet ist und das Endstück E₁ mit dem Elektrodenträger (14) des Handinstruments koppelbar ist.

7. Elektrode (16) für ein elektrochirurgisches Handinstrument nach einem der vorherigen Ansprüche, **dadurch gekennzeichnet, dass** sich der Draht aus mindestens drei Stücken zusammensetzt, nämlich einem ersten Endstück E₁ und einem zweiten Endstück E₂ und mindestens einem Mittelstück MST, wobei das Mittelstück MST zwischen den beiden Endstücken E₁ und E₂ angeordnet ist und die Endstücke E₁ und E₂ mit dem Elektrodenträger (14) des Handinstruments koppelbar sind.

8. Elektrode (16) für ein elektrochirurgisches Handinstrument nach Anspruch 6 oder 7, **dadurch gekennzeichnet, dass** das Mittelstück MST des Drahtes einen Kern (27) aus dem Material M₂ und eine umhüllende äußere Schicht (28) aus dem Material M₁ aufweist oder dass das Material M₁ den Kern (27) des Mittelstücks MST bildet und das Material M₂ die umhüllende äußere Schicht (28).

9. Elektrode (16) für ein elektrochirurgisches Handinstrument nach einem der Ansprüche 6 bis 8, **dadurch gekennzeichnet, dass** sich das Mittelstück MST des Drahtes schalenartig aus mehreren Schichten (28) der Materialien M₁ und M₂ zusammensetzt, wobei sich die Materialien M₁ und M₂ gegenseitig umschließen und der Kern (27) des Drahtes aus dem Material M₁ oder M₂ gebildet ist.

10. Elektrode (16) für ein elektrochirurgisches Handinstrument nach einem der Ansprüche 6 bis 9, **dadurch gekennzeichnet, dass** das mindestens eine Endstück E₁, bzw. die Endstücke E₁ und E₂ aus einem elektrisch leitfähigen oder einem elektrisch nicht leitfähigen Material M₃ gebildet sind.

11. Elektrode (16) für ein elektrochirurgisches Handinstrument nach einem der Ansprüche 6 bis 10, **dadurch gekennzeichnet, dass** die Endstücke E₁ und E₂ jeweils mit einem ihrer Enden (25, 26) mit dem Mittelstück MST elektrisch leitend sowie mechanisch verbunden sind und/oder
dass eines der Endstücke E₁ oder E₂ jeweils mit einem ihrer Enden (25, 26) mit dem Mittelstück MST elektrisch isoliert sowie mechanisch verbunden sind und/oder
dass die Endstücke E₁ und/oder E₂ mit dem Mittelstück MST verklebt, verschweißt, verklemmt, zusammengesteckt oder mittels einer Hülse (29) vercrimpt oder verschweißt sind.

12. Elektrode (16) für ein elektrochirurgisches Handinstrument nach Anspruch 11, **dadurch gekennzeichnet, dass** das Mittelstück MST aus dem Material M₁ oder M₂ und die Hülse (29) komplementär dazu aus dem Material M₂ oder M₁ ausgebildet ist.

13. Elektrode (16) für ein elektrochirurgisches Handinstrument nach einem der vorherigen Ansprüche, **dadurch gekennzeichnet, dass** der Draht einen Durchmesser von 0,2 mm bis 1 mm, vorzugsweise 0,28 mm, aufweist und/oder dass die den Kern (27) umhüllende äußere Schicht (28) oder die Hülse (29) eine Schichtdicke von 3 µm bis 0,2 mm, insbesondere 0,01 mm bis 0,1 mm, vorzugsweise 0,05 mm, aufweist.

14. Elektrode (16) für ein elektrochirurgisches Handinstrument nach einem der vorherigen Ansprüche, **dadurch gekennzeichnet, dass** es sich bei dem Material M₁ um Platin, eine Platinlegierung, wie beispielsweise Platin-Iridium oder Platin-Wolfram, handelt und/oder bei dem Material M₂ um Wolfram, Tantal oder Molybdän und/oder das Material M₃ Stahl, Edelstahl oder Kupfer oder eine Kupferlegierung oder ein elektrischer Isolator ist.

15. Elektrochirurgisches Handinstrument, insbesondere ein Resektoskop (10), mit einer Elektrode (16) nach mindestens einem der Ansprüche 1 bis 14.
